# EUROPEAN PATENT APPLICATION

(11) **EP 4 796 639 A1**
(43) Date of publication of application: **26.08.2026**
(21) Application number: 24880066.6
(22) Date of filing: 14.10.2024
(51) Int. Cl.: C12N 15/67, A61K 48/00, A61K 39/00

(54) **RNA STRUCTURES FOR IMPROVING PROTEIN TRANSLATION EFFICIENCY, mRNA STRUCTURES COMPRISING SAME, AND USES THEREOF**

(30) Priority: 16.10.2023 KR 20230137610
(71) Applicant: Korea Research Institute of Bioscience and Biotechnology, Daejeon 34141 (KR)
(72) Inventor: KIM, Tae-Don, Daejeon 34141 (KR); KIM, Seok-Min, Daejeon 34141 (KR)
(74) Representative: KATZAROV S.A.
(86) International application number: PCT/KR2024/015540
(87) International publication number: WO 2025/084729

(57) **Abstract**

The present invention relates to: an RNA structure for improving the efficiency of non-naturally occurring protein translation, the structure comprising a nucleic acid sequence that comprises two or more stem structures and two or more loop structures, the nucleic acid sequence being a virus-derived internal ribosome entry site(IRES); and a translation-triggering RNA(ttRNA), which is an mRNA structure comprising same. The RNA structure according to the present invention has high RNA stability and protein expression efficiency, low immunogenicity, and can stably express a target protein in a cell for a long time even if various target proteins, various introduction cells and introduction methods are used. Therefore, the mRNA structure comprising the RNA structure having improved protein translation efficiency of the present invention can be effectively utilized in various application fields related to target protein expression, such as the development of therapeutic agents and vaccines.

## Description

### [Technical Field]

### [Cross-reference to related application]

The present application claims the benefit of priority based on Korean Patent Application No. 10-2023-0137610 filed on October 16, 2023, the entire contents of which are incorporated as part of this specification.

### [Technical Field]

The present invention relates to RNA structures for improving protein translation efficiency, mRNA structures comprising the same, and uses thereof.

### [Background Art]

Nucleic acid-based therapy and vaccine development technologies have demonstrated insufficient efficacy as therapeutic agents due to insufficient transcriptional and translational efficiencies when applied to target organisms beyond the cellular level, and this has been pointed out as a limitation of nucleic acid-based therapeutic agent development. Therefore, enhancing the intracellular and extracellular expression capacity of antigenic proteins for the treatment of infectious diseases is one of the essential requirements for the development of pharmaceuticals using artificial nucleic acid molecules. Furthermore, in the field of nucleic acid-based therapy and vaccine development, securing mRNA sequences with high stability and translation efficiency is emerging as an essential requirement for mRNA-based therapeutic agents.

Meanwhile, mRNA is RNA that transmits genetic information from DNA to ribosome and expresses proteins through the translation process mediated by mRNA, and mRNA vaccines refer to pharmaceuticals used for the prevention and treatment of cancer, infectious diseases, and autoimmune diseases, utilizing proteins encoded by mRNA as antigens. mRNA has the following advantages: because it occurs in the cytoplasm, the probability of mutations caused by infection or genomic DNA insertion is lower; because various modifications are possible within the cytoplasm, it can increase intracellular stability by controlling the half-life or increasing the amount of protein being translated; and because it is easy to perform in vitro experiments, rapid development and GMP production are possible, and mass production is easy. Therefore, mRNA therapeutic agents have advantages that surpass DNA therapeutic agents or viral therapeutic agents in terms of stability, efficiency, and productivity.

However, these mRNA vaccines require multifaceted research, given that technical elements are essential for delivery into the cytoplasm due to the instability of RNA molecules, and that damage to RNA molecules or a decrease in translation efficiency caused by excessive innate immune responses must be minimized after RNA molecules are delivered to the cytoplasm. In particular, the immune-activating properties of fundamentally naked mRNA that induce stimulation of adjuvants such as toll-like receptor agonists (TLR agonists) are known to inhibit the expression of mRNA antigens in sufficient quantities to exhibit pharmacological activity because, conversely, they interfere with the transcriptional signaling pathway of mRNA. To solve these problems, active attempts are underway to manufacture vaccines using modified mRNA with removed or weakened immunogenicity. However, in this case, due to the low immunogenicity of the mRNA, there are limitations in inducing effective humoral and/or cellular immune responses.

Under this technical background, it is important to develop mRNA structures that stably express target proteins while possessing low immunogenicity, but research in this area is still insufficient.

### [Disclosure]

### [Technical Problem]

The present invention aims to provide an RNA structure for improving the efficiency of non-naturally occurring protein translation.

Further, the present invention aims to provide an mRNA structure capable of effectively expressing a target protein.

Further, the present invention aims to provide a recombinant vector capable of effectively expressing a target protein.

Further, the present invention aims to provide a transformant capable of effectively expressing a target protein.

Further, the present invention aims to provide a pharmaceutical composition for the prevention or treatment of infectious diseases comprising the mRNA structure; the recombinant vector; or the transformant.

Further, the present invention aims to provide a pharmaceutical composition for the prevention or treatment of non-infectious diseases comprising the mRNA structure; the recombinant vector; or the transformant.

In addition, the present invention aims to provide a vaccine composition comprising the mRNA structure; the recombinant vector; or the transformant.

### [Technical Solution]

To achieve the above objects, one aspect of the present invention provides an RNA structure for improving the efficiency of non-naturally occurring protein translation, comprising a nucleic acid sequence that comprises two or more stem structures; and two or more loop structures, wherein the nucleic acid sequence is a virus-derived internal ribosome entry site(IRES).

Further, to achieve the above objects, another aspect of the present invention provides an mRNA structure comprising (a) a region encoding a target protein or peptide, and (b) the RNA structure downstream of the region encoding the target protein or peptide.

Further, to achieve the above objects, another aspect of the present invention provides a recombinant vector comprising a nucleotide sequence corresponding to the nucleotide sequence of the mRNA structure or a DNA nucleotide sequence complementary thereto.

Further, to achieve the above objects, another aspect of the present invention provides a transformant in which the mRNA structure is introduced into a host cell.

Further, to achieve the above objects, another aspect of the present invention provides a pharmaceutical composition for the prevention or treatment of infectious diseases comprising the mRNA structure; the recombinant vector; or the transformant.

Further, to achieve the above objects, another aspect of the present invention provides a pharmaceutical composition for the prevention or treatment of non-infectious diseases comprising the mRNA structure; the recombinant vector; or the transformant.

In addition, to achieve the above objects, another aspect of the present invention provides a vaccine composition comprising the mRNA structure; the recombinant vector; or the transformant.

### [Advantageous Effects]

The RNA structure according to the present invention has high RNA stability and protein expression efficiency, and low immunogenicity, and can stably express a target protein in a cell for a long time even if various target proteins, various introduction cells and introduction methods are used. In addition, the mRNA structure comprising the RNA structure having improved protein translation efficiency of the present invention can be effectively utilized in various application fields related to target protein expression, such as the development of therapeutic agents and vaccines.

However, the effects of the present invention are not limited to those mentioned above, and other effects not mentioned will be clearly understood by those skilled in the art from the description below.

### [Description of Drawings]

FIG. 1A shows mRNA structures constructed by combining a GFP-encoding region, a 5'-β-globin UTR region, a BGH region, and an EMCV-derived IRES region.
FIG. 1B shows the expression level of GFP mRNA measured using the mRNA structures of FIG. 1A.
FIG. 1C shows the expression level of GFP protein measured using the mRNA structures of FIG. 1A.
FIG. 2A shows mRNA structures constructed by combining a region encoding a CAR protein, a 5'-β-globin UTR region, a BGH region, and an EMCV-derived IRES region.
FIG. 2B shows the expression level of CAR mRNA measured using the mRNA structures of FIG. 2A.
FIG. 2C shows the expression level of CAR protein measured using the mRNA structures of FIG. 1A.
FIG. 3A shows mRNA structures constructed by combining a region encoding a SARS-CoV-2 spike protein, a 5'-β-globin UTR region, a BGH region, an EMCV-derived IRES region, and a poly-A region, as well as a structure of the COVID-19 mRNA vaccine BNT162b2 sold by Pfizer company.
FIG. 3B shows the expression levels of SARS-CoV-2 spike mRNA measured using the mRNA structures and BNT162b2 of FIG. 3A.
FIG. 3C shows the expression level of SARS-CoV-2 spike protein measured using the mRNA structures and BNT162b2 of FIG. 3A.
FIG. 4 shows a schematic diagram of the process of simplifying the secondary structure of the IRES region.
FIG. 5A shows mRNA structures containing a region encoding the spike protein of SARS-CoV-2 and an EMCV-derived IRES region or an EMCV-derived modified IRES region.
FIG. 5B shows the expression level of SARS-CoV-2 spike protein measured using the mRNA structures of FIG. 5A and BNT162b2.
FIG. 5C shows the stability of SARS-CoV-2 spike mRNA measured using the mRNA structures of FIG. 5A and BNT162b2.
FIG. 5D shows the results of measuring the immunogenicity of the mRNA structures of FIG. 5A and BNT162b2.
FIG. 5E shows mRNA structures in which modified IRES regions are introduced between the BGH region and the poly-A region.
FIG. 5F shows the GFP expression level and mRNA stability measured using the mRNA structures of FIG. 5E.
FIG. 6 shows a schematic diagram of the process of simplifying the secondary structure of the IRES region of poliovirus, foot-and-mouth disease virus, and hepatitis C virus.
FIG. 7A shows structures of an mRNA structure containing a region encoding the spike protein of SARS-CoV-2 and the EMCV-derived modified IRES region, an mRNA structure containing the poliovirus-derived modified IRES region, an mRNA structure containing the foot-and-mouth disease virus-derived modified IRES region, and an mRNA structure containing the hepatitis C virus-derived modified IRES region, as well as a structure of the COVID-19 mRNA vaccine BNT162b2 sold by Pfizer company.
FIG. 7B shows the expression level of SARS-CoV-2 spike protein measured using the mRNA structures and BNT162b2 of FIG. 7A.

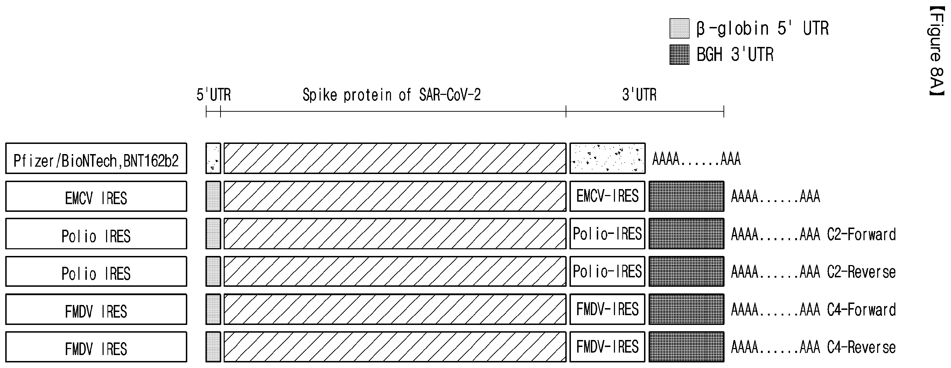

FIG. 8A shows structure of an mRNA structure containing a region encoding the spike protein of SARS-CoV-2 and the EMCV-derived modified IRES region, an mRNA structure containing the poliovirus-derived modified IRES region and an mRNA structure containing the poliovirus-derived modified IRES region in the opposite direction, an mRNA structure containing the foot-and-mouth disease virus-derived modified IRES region and an mRNA structure containing the foot-and-mouth disease virus-derived modified IRES region in the opposite direction, and the structure of the COVID-19 mRNA vaccine BNT162b2 sold by Pfizer company.
FIG. 8B shows the expression level of SARS-CoV-2 spike protein measured using the mRNA structures and BNT162b2 of FIG. 8A.
FIG. 9A shows BGH UTR regions (SEQ ID NOs: 14 to 17) constructed to consist of 91, 55, 36, and 18 nucleotides, respectively, by changing the length of the BGH UTR region of SEQ ID NO: 9, which consists of 215 nucleotides, and mRNA structures containing each of these.
FIG. 9B shows the expression level of GFP protein measured using the mRNA structures of FIG. 9A.
FIG. 10A shows mRNA structures containing a region encoding the spike protein of SARS-CoV-2, wherein a modified EMCV IRES region is located upstream of a BGH UTR region, and a modified EMCV IRES region is located upstream of an optimized BGH UTR region consisting of 36 nucleotides, and the structure of mRNA-1273 sold by Moderna company.
FIG. 10B shows the expression level of SARS-CoV-2 spike protein measured using the mRNA structures and mRNA-1273 of FIG. 10A.
FIG. 11A shows the structure of mRNA containing a region encoding the PD-L1 CAR protein and a modified EMCV IRES region located upstream of an optimized BGH UTR region consisting of 36 nucleotides, and the structure of mRNA-1273 sold by Moderna company.
FIG. 11B shows the expression level of PD-L1 CAR protein measured using the mRNA structure and mRNA-1273 of FIG. 11A.
FIG. 12 shows the mRNA structure containing the region encoding the spike protein of SARS-CoV-2 and the EMCV-derived modified IRES region, and the structure of the COVID-19 mRNA vaccine BNT162b2 sold by Pfizer company.
FIG. 13A shows the expression level of SARS-CoV-2 spike protein measured after introducing the mRNA structure and BNT162b2 of FIG. 12 into epithelial cells HEK-293T.
FIG. 13B shows the expression levels of SARS-CoV-2 spike protein measured after introducing the mRNA structure and BNT162b2 of FIG. 12 into muscle cells c2c12.
FIG. 14A shows the expression level of SARS-CoV-2 spike protein measured after introducing the mRNA structure and BNT162b2 of FIG. 12 into epithelial cells HEK-293T using liposomes.
FIG. 14B shows the expression level of SARS-CoV-2 spike protein measured after introducing the mRNA structure and BNT162b2 of FIG. 12 into epithelial cells HEK-293T using LNP.

### [Best Mode]

The present invention will be described in detail below.

### 1. RNA structure

One aspect of the present invention provides an RNA structure for improving the efficiency of non-naturally occurring protein translation, comprising a nucleic acid sequence that comprises two or more stem structures and two or more loop structures, wherein the nucleic acid sequence is a virus-derived internal ribosome entry site(IRES).

The RNA structure of the present invention can significantly increase the translation efficiency of non-naturally occurring proteins. This RNA structure comprising the nucleic acid sequence of the present invention may be referred to as translation-triggering RNA (ttRNA).

In the present invention, the stem structure and loop structure are structures generated by complementary binding between base pairs in single-stranded DNA or RNA, and are also referred to as hairpin structures.

In the present invention, the nucleic acid sequence may be a modified virus-derived internal ribosome entry site (IRES) or may be artificially produced from the sequence.

In the present invention, the modification may be at least one selected from the group consisting of variants and modifications of nucleotides constituting the nucleic acid sequence.

In the present invention, the variant of the nucleotide may include the substitution, deletion, insertion, or any combination thereof of one or more nucleotides constituting the nucleic acid sequence, and the nucleic acid sequence itself may be changed through the variant. Meanwhile, the variant may be used interchangeably with mutation.

In addition, in the present invention, the modification of the nucleotide means that one or more nucleotides constituting the nucleic acid sequences are chemically changed, and the modified nucleotide may include a modified base, a modified pentose, a modified phosphate, a modified base-to-base linkage, or any combination thereof.

The modified bases may be diaminopurine, isocytosine, isoguanine, pseudoisocytosine, N9-(2-amino-6-chloropurine), N9-(2,6-diaminopurine), N9-(7-deazaguanine), N9-(7-deaza-8-aza-guanine) and N8-(7-deaza-8-aza-adenine), 5-propynyl-uracil, 2-thio-5-propynyl-uracil, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueosin, inosine, N6-isopentenyladenine, 1-methylguanine, 1-methylinosine, 2,2-dimethylguanine, 2-methyladenine, 2-methylguanine, 2-thiothimine, 2-aminopurine, 3-methylcytosine, 5-methylcytosine, N6-adenine, 7-methylguanine, 5-methylaminomethyluracil, 5-methoxyaminomethyl-2-thiouracil, beta-D-mannosylqueosin, 5'-methoxycarboxymethyluracil, 5-methoxyuracil, 2-methylthio-D46-isopentenyladenine, uracil-5-oxyacetic acid (v), wybutoxosine, pseudouracil, queosin, 2-thiocytosine, 5-methyl-2-thiouracil, 2-thiouracil, 4-thiouracil, 5-methyluracil, uracil-5-oxyacetate methyl ester, uracil-5-oxyacetate (v), 5-methyl-2-thiouracil, 3-(3-amino-3-N-2-carboxypropyl)uracil, (acp3)w, oligonucleotide phosphoramidate, nitropyrrole, nitroindole, acylic 5-nitroindole, 4-nitropyrazole, 4-nitroimidazole, C-5-propynyl-C, C-5-propynyl-U or 2-aminoadenine.

The modified pentose may be a lock nucleic acid (LNA) analog (e.g., Bz-A-LNA, 5-Me-Bz-C-LNA, dmf-G-LNA or T-LNA, etc.) or a 2'- or 3'- variant (wherein the 2'- or 3'-position is hydrogen, hydroxy, alkoxy (e.g., methoxy, ethoxy, allyloxy, isopropoxy, butoxy, isobutoxy and phenoxy), azido, amido, alkylamino, fluoro, chloro, or bromo).

The above modified phosphate may be a phosphate analog in which a phosphorus atom exists in a +5 oxidation state and one or more of the oxygen atoms are replaced by a non-oxygen moiety, e.g., sulfur. Another modified phosphate may be a phosphorothioate, phosphorodithioate, phosphoroselenoate, phosphorodiselenoate, phosphoranilothioate, phosphoranilideate, phosphoramidate, or voronophosphate, containing associated counterions, e.g., H⁺, NH4⁺, Na⁺.

The base-to-base linkage may be a phosphate analog, an achiral analog, an uncharged inter-subunit linkage, or an uncharged morpholino-based polymer having an achiral inter-subunit linkage. Some base-to-base linkage analogs may be morpholidates, acetals, or polyamide-linked heterocycles. Additionally, if the base is a peptide nucleic acid (PNA), the modified inter-base linkage portion may be a 2-aminoethylglycinamide backbone polymer.

In the present invention, through the modification, a conventional virus-derived IRES sequence having a long and complex secondary structure can be simplified into a short nucleic acid sequence comprising two or more stem structures and two or more loop structures. After the simplification, the IRES sequence may further undergo a process of optimization by selecting a region having a Clover leaf structure. The selection or optimization may refer to a process of deriving a nucleic acid sequence in which, compared to RNA structures containing the entire IRES sequence, the protein expression level is maintained while mRNA stability is significantly increased and immunogenicity is reduced, resulting in a significantly increased overall protein expression efficiency.

In the present invention, the "IRES (Internal Ribosome Entry Site)" refers to an internal ribosome entry site or a ribosome binding site, and it is known that it forms a loop structure on mRNA to induce the initiation of translation through a mechanism independent of the Cap.

In the present invention, the virus may be Encephalomyocarditis virus (EMCV), Poliovirus, Foot-and-mouth disease virus (FMDV), or Hepatitis C virus (HCV), but is not necessarily limited thereto, and any virus including the IRES region may be used without limitation.

In the present invention, the non-naturally occurring protein refers to a protein that is not normally expressed by a cell or organism, and may include all non-naturally occurring proteins that a person skilled in the art intends to express or produce in large quantities. For example, the non-naturally occurring protein may be at least one selected from the group consisting of antigens, viral proteins, enzymes, chimeric antigen receptors (CARs), cell receptors, and serum proteins, but is not limited thereto.

In the present invention, the nucleic acid sequence may include 2 to 8 stem structures; and 2 to 8 loop structures. For example, the nucleic acid sequence may include 3 to 7, 3 to 6, 3 to 5, 2 to 7, 2 to 6, or 2 to 5 stem structures, and may include 3 to 7, 3 to 6, 3 to 5, 2 to 7, 2 to 6, or 2 to 5 loop structures.

In addition, in the present invention, the RNA structures may bind to eIF (eukaryotic translation initiation factor). The "eIF" is a protein or protein complex involved in the protein translation initiation step of eukaryotes and is known to perform the function of stabilizing the formation of ribosome complexes around the start codon. The eIF may be, for example, eIF1, eIF1A, eIF2, eIF3, eIF4, eIF5, eIF5A, eIF5B, or eIF6, but is not limited thereto, and any eIF widely known in the field may be included without limitation.

In the present invention, the nucleic acid sequence may be composed of any one of the nucleotide sequences of SEQ ID NO: 1 to SEQ ID NO: 7.

The nucleotide sequences of SEQ ID NO: 1 to SEQ ID NO: 7 are nucleotide sequences in which a region having a Clover leaf structure is selected from the entire IRES sequence and simplified into a short sequence, and represent modified IRES sequences in which the amount of protein translation is similar to that when the entire IRES sequence is used, while mRNA stability is significantly increased and immunogenicity is reduced, resulting in a significantly increased overall protein expression efficiency.

The nucleotide sequences of SEQ ID NO: 1 to SEQ ID NO: 7 each comprise a polynucleotide having a nucleotide sequence substantially the same as the nucleotide sequence of SEQ ID NO: 1 to SEQ ID NO: 7, a variant thereof, or an active fragment thereof. The polynucleotide having substantially the same nucleotide sequence means a polynucleotide comprising a nucleotide sequence having sequence homology of at least about 70%, at least about 75%, at least about 80%, at least about 85%, at least about 90%, at least about 92%, at least about 95%, at least about 97%, at least about 98%, or at least about 99% with respect to the nucleotide sequences of SEQ ID NO: 1 to SEQ ID NO: 7, respectively.

### 2. mRNA structure

Another aspect of the present invention provides an mRNA structure comprising (a) a region encoding a target protein or peptide, and (b) an RNA structure downstream of the region encoding the target protein or peptide.

The overlapping descriptions are identical to those previously described in '1. RNA structure', so they are omitted.

In the present invention, the target protein or peptide is a protein or peptide that a person skilled in the art intends to express or produce in large quantities, and may include all proteins and peptides intended to be expressed using the mRNA structures of the present invention. For example, in the present invention, the target protein or peptide may be at least one selected from the group consisting of antigens, viral proteins, enzymes, chimeric antigen receptors (CARs), cell receptors, and serum proteins, but is not limited thereto.

In addition, in the present invention, the RNA structure may be included in the 3'-UTR of the mRNA, which is the downstream region of the region encoding the target protein or peptide, and may not be included in the 5'-UTR of the mRNA, which is the upstream region of the region encoding the target protein or peptide.

The mRNA structure of the present invention is intended for use in transformation, preferably transient transformation, which expresses a target protein only for a certain period of time without gene insertion into the chromosomes of a host cell. While it is important for the mRNA to be structurally stable so as to express the protein with high efficiency for a long period, it is ultimately an essential condition for achieving the purpose of transformation that the target protein must be able to express the target protein with high efficiency while fully exhibiting its original function.

Accordingly, the inventors confirmed that when an mRNA structure is constructed using an RNA structure comprising a virus-derived IRES nucleic acid sequence that comprises two or more stem structures; and two or more loop structures to express a protein, mRNA stability and protein translation efficiency are increased while immunogenicity is reduced, allowing for more efficient long-term expression of the target protein. Specifically, in an embodiment of the present invention, it was confirmed that the mRNA structure of the present invention, which includes a modified IRES sequence in the 3'-UTR of the mRNA downstream region of the target protein or peptide, has significantly higher SARS-CoV-2 spike mRNA stability and protein expression efficiency and significantly lower immunogenicity compared to BNT162b2, a COVID-19 mRNA vaccine sold by Pfizer company. Therefore, since the mRNA structure of the present invention can stably express the target protein for a long time within cells, it can be effectively utilized in various application fields related to target protein expression, such as anticancer immunotherapy and vaccine development.

In the present invention, the region encoding the target protein or peptide and the RNA structure are composed of a nucleotide sequence, and the "nucleotide sequence" refers to a polymeric material comprising a plurality of nucleotide units, specifically, a polymer in which a plurality of nucleotide units are connected to each other by phosphodiester bonds of a sugar/phosphate basic backbone, and can be used interchangeably with the terms "polynucleotide," "nucleic acid," and "nucleic acid molecule." The polynucleotide is a biopolymer essential to living organisms and may be RNA or DNA that codes for genetic information through its unique nucleotide sequence. The polynucleotide may be isolated, artificially synthesized, or non-naturally occurring or engineered, wherein "non-naturally occurring or engineered" refers to a state created by applying artificial modifications rather than the state in which it exists naturally. Here, the artificial modification may be intended to enhance the expression of a target antigen in cells by mimicking the structure of natural mRNA, specifically mature mRNA.

In the present invention, "mRNA (Messenger RNA)" refers to RNA that is transcribed from a DNA template and transmits the genetic information of DNA to ribosomes in the cytoplasm. The transcription process in eukaryotic organisms takes place within the nucleus of the cell and includes the process of processing premature RNA. Specifically, this process is referred to as post-transcriptional modification and includes processes such as splicing, 5'capping, polyadenylation, and export from the nucleus or mitochondria. As a result of these processes, mature mRNA containing a nucleotide sequence that can be translated into the amino acid sequence of a specific peptide or protein is produced. Generally, the mature mRNA may optionally include a 5'-cap, a 5'UTR, an open reading frame, a 3'UTR, and a poly A-tail. The mRNA may be synthesized according to any of several known methods, for example, the mRNA may be synthesized via in vitro transcription (IVT).

In the present invention, the "5'Cap" is a structure located in the 5' terminal region that affects the stability and expression efficiency of the polynucleotide, and can generally be formed by a modified nucleotide, particularly a derivative of a guanine nucleotide.

In addition, in the present invention, "Poly A tail" is a structure located in the 3' terminal region that delays the degradation process of RNA exo-nuclease and extends the stability and in vivo half-life of polynucleotides, thereby affecting expression efficiency, and can generally be formed by a plurality of adenine nucleotide sequences. In one embodiment, the poly A-tail may consist of 20 to 200 adenines, for example, 20 to 190, 20 to 170, 20 to 150, 20 to 130, 20 to 110, 20 to 90, 20 to 70, 20 to 50, 20 to 30, 30 to 190, 30 to 170, 30 to 150, 30 to 130, 30 to 110, 30 to 90, 30 to 70, or 30 to 50 repeating adenine nucleotide sequences. Additionally, the poly A-tail may be composed of a plurality of units connected by a linker.

The mRNA structure of the present invention may further include a 5'-UTR (untranslated region) region of β-globin upstream of the region encoding the target protein or peptide.

Specifically, in the present invention, the 5'-UTR region of the β-globin may have a nucleotide sequence of SEQ ID NO: 8, but is not limited thereto.

Additionally, the nucleotide sequence of SEQ ID NO: 8 comprises a polynucleotide having a nucleotide sequence substantially identical to the nucleotide sequence of SEQ ID NO: 8, a variant thereof, or an active fragment thereof. The polynucleotide having a substantially identical nucleotide sequence means a polynucleotide comprising a nucleotide sequence having sequence homology of at least about 70%, at least about 75%, at least about 80%, at least about 85%, at least about 90%, at least about 92%, at least about 95%, at least about 97%, at least about 98%, or at least about 99% with respect to the nucleotide sequence of SEQ ID NO: 8.

In addition, the mRNA structure of the present invention may further include a 3'-UTR region of BGH (bovine growth hormone) downstream of the region encoding the target protein or peptide, and the 3'-UTR region of BGH may be included downstream of the modified IRES region.

Specifically, in the present invention, the 3'-UTR region of the BGH may have a nucleotide sequence of SEQ ID NO: 9, but is not limited thereto.

Additionally, the nucleotide sequence of SEQ ID NO: 9 comprises a polynucleotide having a nucleotide sequence substantially the same as the nucleotide sequence of SEQ ID NO: 9, a variant thereof, or an active fragment thereof. The polynucleotide having a substantially identical nucleotide sequence means a polynucleotide comprising a nucleotide sequence having sequence homology of at least about 70%, at least about 75%, at least about 80%, at least about 85%, at least about 90%, at least about 92%, at least about 95%, at least about 97%, at least about 98%, or at least about 99% with respect to the nucleotide sequence of SEQ ID NO: 9.

In addition, in the present invention, the 3'-UTR region of the BGH may be a shortened nucleotide sequence of SEQ ID NO: 9 consisting of 215 nucleotides, with a certain number of nucleotides deleted. For example, in the present invention, the 3'-UTR region of the BGH may be a nucleotide sequence consisting of 91 nucleotides (SEQ ID NO: 14), a nucleotide sequence consisting of 55 nucleotides (SEQ ID NO: 15), a nucleotide sequence consisting of 36 nucleotides (SEQ ID NO: 16), or a nucleotide sequence consisting of 18 nucleotides (SEQ ID NO: 17), and in particular, a nucleotide sequence consisting of 36 nucleotides (SEQ ID NO: 16).

Specifically, in one embodiment of the present invention, it was confirmed that when a modified IRES region is introduced upstream of a BGH UTR region, the target protein expression level is similar to that when it is introduced between a BGH UTR region and a poly-A region, but the mRNA stability is significantly higher. In addition, in another embodiment of the present invention, mRNA structures were constructed by varying the length of a BGH UTR region of SEQ ID NO: 9 consisting of 215 nucleotides, and as a result of comparing the target protein expression levels of each structure, it was confirmed that the GFP expression level of an mRNA structure containing a BGH UTR region (SEQ ID NO: 16) consisting of 36 nucleotides was significantly higher than that of the existing BGH UTR region of SEQ ID NO: 9.

Furthermore, in another embodiment of the present invention, the modified IRES region as described above is introduced upstream of the BGH UTR region, and it was confirmed that the level of target protein expression in mRNA structures containing a BGH UTR region (SEQ ID NO: 16) composed of 36 nucleotides is up to approximately 4.8 times higher than that of Moderna company's mRNA-1273 structure. By deriving the optimal location for introducing the modified IRES region and the optimal sequence of the BGH UTR region, an optimal mRNA structure with excellent mRNA stability and target protein expression ability was derived.

In the present invention, the "UTR (untranslated region)" is also referred to as an untranslated region or an unreadable region, and refers to a part of the mRNA chain that does not serve as a template for a protein gene, i.e., a part that is not translated. It is a term referring to the unreadable region of mRNA, and generally, the upstream of the region encoding a protein is referred to as the 5'-UTR, and the downstream as the 3'-UTR.

In the present invention, "BGH (bovine growth hormone)" refers to a bovine growth factor and signifies the polyadenylation signal of the bovine growth factor. The BGH region includes a polyadenylation signal together with a 5'-β-globin UTR region.

The mRNA structure of the present invention may further include, without limitation, regions capable of enhancing the protein expression rate from the mRNA. For example, it may further include a region that induces ribosomes, a region related to the initiation or promotion of translation of mRNA, a region related to the transport of mRNA out of the nucleus, a region related to binding to the endoplasmic reticulum membrane, a region containing an endoplasmic reticulum holding signal (ER holding signal) sequence, or a region containing an endoplasmic reticulum signal sequence.

### 3. Recombinant vector and transformant

Another aspect of the present invention provides a recombinant vector comprising a nucleotide sequence corresponding to the nucleotide sequence of the mRNA structure or a DNA nucleotide sequence complementary thereto.

The overlapping descriptions are identical to those previously described in '1. RNA structure' and '2. mRNA structure', so they are omitted.

The recombinant vector of the present invention is an expression vector, wherein the expression vector refers to a gene construct comprising an essential regulatory element operably linked to an insert so as to express the insert within a cell.

In the present invention, the "operably linked" means a state in which a region encoding a target protein is connected to a regulatory element in a manner that allows the expression of a nucleotide sequence.

In the present invention, the expression vector may be prepared and purified using standard recombinant DNA technology. The type of the expression vector is not particularly limited as long as it functions to express a desired gene and produce a desired protein in various host cells, including prokaryotic and eukaryotic cells; however, it may be a vector capable of producing a large amount of target protein while possessing a promoter exhibiting potent activity and strong expression power, but is not limited thereto. For example, the expression vector may be a vector composed of plasmids, cosmids, artificial chromosomes, liposomes, retrovirus, adenovirus, adenovirus-associated virus (AAV), vaccinia virus, herpes virus, lentivirus, or spumavirus, but is not limited thereto.

In addition, the expression vector may include, but is not limited to, a promoter, a start codon, a gene encoding a target protein, and a stop codon terminator. In addition, it may appropriately include, but is not limited to, DNA encoding a signal peptide, an enhancer sequence, non-translating regions of the 5' and 3' of the desired gene, a selection marker region, or a replicable unit.

The recombinant vector of the present invention comprises an mRNA structure comprising a virus-derived IRES nucleic acid sequence that comprise two or more stem structures and two or more loop structures, thereby enabling stable expression of a target protein in a transformant for a long time, and thus can be effectively utilized in various application fields related to target protein expression and production, such as anticancer immunotherapy and vaccine development.

In addition, another aspect of the present invention provides a transformant in which the mRNA structure is introduced into a host cell.

The transformant refers to a host cell into which foreign genetic material has been introduced by transfection or transduction. In the present invention, the transformant may refer to a host cell into which a recombinant vector, comprising a nucleotide sequence corresponding to the mRNA structure of the present invention or the nucleotide sequence of the mRNA structure of the present invention, or a DNA nucleotide sequence complementary thereto, is introduced.

The transformant of the present invention can effectively express a target protein by introducing the mRNA structure or recombinant vector of the present invention and transforming it.

In the present invention, the term "introduction" refers to the process of transforming a host cell by injecting or introducing foreign genetic material into the host cell, and the host cell may be, for example, an immune cell, an epithelial cell, a muscle cell, a kidney cell, a tumor cell, etc.

The foreign genetic material may be introduced into a cell by physical, chemical, or biological means. The physical means may include a gene gun, calcium phosphate precipitation, lipofection, particle bombardment, microinjection, electroporation, etc., and the chemical means may include colloidal dispersion systems, such as macromolecular complexes, nanocapsules, microspheres, beads, and lipid-based systems including oil-in-water emulsions, micelles, LNPs, mixed micelles, and liposomes. Additionally, the biological means may include the use of DNA or RNA vectors.

In the present invention, the liposome or LNP comprises cationic lipids, non-cationic lipids, or neutral lipids (phospholipids, etc.), and additionally may include other lipids such as PEG (polyethylene glycol) or cholesterol, or cell-permeable peptides, etc. Specific examples of the cationic lipid may include ALC-0315([(4-hydroxybutyl)azanediyl]di(hexane-6,1-diyl) bis(2-hexyldecanoate)), SM-102(9-Heptadecanyl 8-{(2-hydroxyethyl)[6-oxo-6-(undecyloxy)hexyl]amino}octanoate), N,N-dioleyl-N,N-dimethyIammonium chloride (DODAC), N-(l-(2,3-dioleyloxy)propyl)-N,N,N-trimethylammonium chloride(DOTMA), N-(l-(2,3-dioleoyloxy)propyl)-N,N,N- trimethylammonium chloride (DOTAP), N,N-dimethyl-(2,3-dioleyloxy)propylamine (DODMA), N,N-distearyl-N,N-dimethylammonium bromide(DDAB), 1,2-dilinoleyloxy-N,N-dimethylaminopropane(DLinDMA), 1,2-dilinolenyloxy-N,Ndimethylaminopropane (DLenDMA), DLin-KC2-DMA, (6Z,9Z,28Z,31Z)-Heptatriaconta-6,9,28,31-tetraen-19-yl 4-(dimethylamino)butanoate (DLinMC3-DMA, CAS 1224606-06-7), ((4-hydroxybutyl)azanediyl)bis(hexane-6,1-diyl)bis(2-hexyldecanoate), etc., but are not limited thereto.

In this specification, transfection and transduction may be used interchangeably, but both can be interpreted in a broad sense as the delivery of foreign genetic material to a host cell to transform the host cell.

Meanwhile, the above transfection can be classified as a stable transfection if the injected foreign genetic material is inserted into the chromosomal DNA of the host cell or is in a form capable of independent replication, and as a transient transfection if it is not.

### 4. Pharmaceutical composition for prevention or treatment of infectious diseases or non-infectious diseases

Another aspect of the present invention provides a pharmaceutical composition for the prevention or treatment of infectious diseases comprising the mRNA structure; the recombinant vector; or the transformant.

In addition, another aspect of the present invention provides a pharmaceutical composition for the prevention or treatment of non-infectious diseases comprising the mRNA structure; the recombinant vector; or the transformant.

The overlapping descriptions are the same as those previously described in '*1. RNA structure'* through '*3. Recombinant vector and transformant'*, so they are omitted.

In the present invention, the infectious diseases may be at least one selected from the group consisting of sepsis, septic shock, Severe Acute Respiratory Syndrome Coronavirus (SARS-CoV) infection, Middle East Respiratory Syndrome (MERS), salmonellosis, food poisoning, typhoid fever, paratyphoid fever, Systemic Inflammatory Response Syndrome (SIRS), Multiple Organ Dysfunction Syndrome (MODS), pneumonia, pulmonary tuberculosis, tuberculosis, common cold, influenza, respiratory infection, rhinitis, nasopharyngitis, otitis media, bronchitis, lymphadenitis, parotitis, lymphadenopathy, cheilitis, stomatitis, arthritis, myositis, dermatitis, vasculitis, gingivitis, periodontitis, keratitis, conjunctivitis, wound infection, peritonitis, hepatitis, osteomyelitis, cellulitis, meningitis, encephalitis, brain abscess, encephalomyelitis, cerebral meningitis, osteomyelitis, nephritis, carditis, endocarditis, enteritis, gastritis, esophagitis, duodenitis, colitis, urinary tractitis, cystitis, vaginitis, cervicitis, salpingitis, infectious erythema, bacterial dysentery, abscess and ulcer, bacteremia, diarrhea, dysentery, gastroenteritis, gastroenteritis, genitourinary abscess, infection of open wounds or wounds, suppurative inflammation, abscess, boil, pyoderma, impetigo, folliculitis, cellulitis, postoperative wound infection, skin laceration syndrome, skin burn syndrome, thrombotic thrombocytopenia, hemolytic uremic syndrome, renal failure, pyelonephritis, glomerulonephritis, nervous system abscess, otitis media, sinusitis, pharyngitis, tonsillitis, mastoiditis, cellulitis, odontogenic infection, dacryocystitis, pleuritis, abdominal abscess, liver abscess, cholecystitis, splenic abscess, pericarditis, myocarditis, placentaitis, amnioticitis, mastitis, puerperal fever, toxic shock syndrome, Lyme disease, gas gangrene, atherosclerosis, Mycobacterium avium syndrome (MAC), enterohemorrhagic E. coli (EHEC) infection, enteropathogenic E. coli (EPEC) infection, enteroinvasive E. coli (EIEC) infection, methicillin-resistant Staphylococcus aureus (MRSA) infection, vancomycin-resistant Staphylococcus aureus (VRSA) infection, and listerosis.

In addition, in the present invention, the non-infectious diseases may be at least one selected from the group consisting of cancer, autoimmune diseases, non-infectious inflammatory diseases, neurodegenerative diseases, heart diseases, stroke, diabetes, metabolic diseases, chronic kidney diseases, and chronic respiratory diseases.

In the present invention, the "prevention" refers to any act of suppressing a specific disease or disorder or delaying its onset by administering a pharmaceutical composition according to the present invention, and the "treatment" refers to any act of improving or beneficially altering the symptoms of a specific disease or disorder by administering a pharmaceutical composition according to the present invention.

Furthermore, in the present invention, "administration" means physically introducing a pharmaceutical composition according to the present invention to an individual using any of the various methods and delivery systems known to a person skilled in the art, and in the present invention, "individual" refers to a subject requiring prevention or treatment of a specific disease or condition, specifically, humans or mammals such as primates, mice, rats, dogs, cats, horses, pigs, rabbits, and cattle.

The pharmaceutical composition of the present invention may be provided with the active ingredient alone, or with one or more pharmaceutically acceptable carriers, excipients, or diluents.

The meaning of the "pharmaceutically acceptable" is that the subject of application (prescription) does not possess toxicity beyond tolerable limits without inhibiting the activity of the active ingredient, and the "carrier" refers to a compound that facilitates the addition of the compound into cells or tissues.

Specifically, the carrier may be, for example, a colloidal suspension, powder, saline solution, lipid, liposome, microsphere, or nano-spherical particle. These may form a complex with or be associated with a delivery means and may be delivered in vivo using a delivery system known in the art, such as lipids, liposomes, microparticles, gold, nanoparticles, polymers, condensation agents, polysaccharides, polyamino acids, dendrimers, saponins, adsorption-enhancing substances, or fatty acids.

When the pharmaceutical composition of the present invention is formulated, it may be prepared using diluents or excipients such as commonly used lubricants, sweeteners, flavorings, emulsifiers, suspending agents, preservatives, fillers, volume expanders, binders, wetting agents, disintegrants, and surfactants. Solid dosage forms for oral administration may include tablets, pills, powders, granules, capsules, etc., and such solid dosage forms may be prepared by mixing at least one excipient, for example, starch, calcium carbonate, sucrose or lactose, gelatin, etc., with the above pharmaceutical composition. In addition, lubricants such as magnesium stearate and talc may also be used in addition to simple excipients. Liquid formulations for oral administration include suspensions, oral liquids, emulsions, syrups, etc., and may contain various excipients, such as humectants, sweeteners, flavorings, and preservatives, in addition to commonly used simple diluents like water and liquid paraffin. Formulations for parenteral administration may include sterile aqueous solutions, non-aqueous solvents, suspensions, emulsions, lyophilized formulations, and suppositories. Propylene glycol, polyethylene glycol, vegetable oils such as olive oil, and injectable esters such as ethyl oleate may be used as non-aqueous solvents and suspensions. Witepsol, macrogol, tween 61, cacao gelatin, laurin gelatin, glycerogelatin, etc. may be used as bases for suppositories, and known diluents or excipients may be used when manufactured in the form of ophthalmic preparations.

Meanwhile, the pharmaceutical composition of the present invention may be appropriately administered to an individual according to the conventional methods, routes of administration, and dosages used in the art, depending on the purpose or need. Examples of routes of administration may include oral, parenteral, subcutaneous, intraperitoneal, intrapulmonary, and nasal administration, and parenteral administration includes intramuscular, intravenous, intra-arterial, intraperitoneal, or subcutaneous administration. In addition, an appropriate dosage and number of administrations may be selected according to methods known in the art, and the amount and number of administrations of the pharmaceutical composition of the present invention actually administered may be appropriately determined by various factors such as the type of symptom to be treated, the route of administration, gender, health status, diet, age and weight of the individual, and the severity of the disease.

The pharmaceutical composition of the present invention is administered in a pharmaceutically effective amount. The "pharmaceutically effective amount" refers to an amount sufficient to suppress or alleviate a disease at a reasonable rate applicable to medical use, and the effective dose level may be determined based on factors including individual type and severity, age, gender, drug activity, sensitivity to the drug, time of administration, route of administration and elimination rate, duration of treatment, concurrently used drugs, and other factors well known in the medical field. The pharmaceutical composition of the present invention may be administered as an individual therapeutic agent or in combination with other therapeutic agents, and may be administered sequentially or simultaneously with conventional therapeutic agents. It may also be administered as a single or multiple doses. It is important to administer an amount that obtains maximum effect with a minimum amount without side effects by taking all of the above factors into consideration, and this can be easily determined by a person skilled in the art. For example, a pharmaceutically effective amount may be 0.5 to 1000 mg/day/kg body weight, and specifically 0.5 to 500 mg/day/kg body weight.

Another aspect of the present invention provides a method for preventing or treating infectious diseases, comprising the step of treating an individual with the mRNA structure; the recombinant vector; or the transformant.

Another aspect of the present invention provides a method for preventing or treating non-infectious diseases, comprising the step of treating an individual with the mRNA structure; the recombinant vector; or the transformant.

Another aspect of the present invention provides the use of the mRNA structure; the recombinant vector; or the transformant for the prevention or treatment of infectious diseases.

Another aspect of the present invention provides the use of the mRNA structure; the recombinant vector; or the transformant for the prevention or treatment of non-infectious diseases.

### 5. Vaccine composition

Another aspect of the present invention provides a vaccine composition comprising the mRNA structure; the recombinant vector; or the transformant.

The overlapping descriptions are the same as those previously described in '*1. RNA structure'* through '*4. Recombinant vector and transformant'*, so they are omitted.

In the present invention, the "vaccine" refers to a substance comprising a component effective in preventing infection or reinfection by the pathogen or antigen, reducing the severity of symptoms or eliminating symptoms, or substantially or completely eliminating a disease caused by the pathogen or antigen by inducing an immune response to a specific antigen in an individual.

The "antigen" refers to a molecule capable of inducing an immune response within a host organism, and in the present invention, the antigen may be at least one selected from the group consisting of cells, viruses, proteins, peptides, nucleic acids, oligonucleotides, carbohydrates, and lipids, and may include all derived from microorganisms including viruses, bacteria, or fungi, and derived from cancer cells.

In the present invention, when the antigen is derived from a virus, the virus may be at least one selected from the group consisting of, for example, influenza virus, coronavirus, flavivirus, calicivirus, respiratory syncytial virus (RSV), rotavirus, parvovirus, picornavirus, pestivirus, rhabdovirus, birnavirus, retrovirus, and herpesvirus, but is not limited thereto.

The vaccine composition of the present invention may be prepared in any suitable, pharmaceutically acceptable formulation. For example, it may be in the form of an immediate-administration solution or suspension, a concentrated stock solution suitable for dilution prior to administration, or a reconstitutable form such as a lyophilized, freeze-dried, or frozen formulation.

The vaccine composition of the present invention may be formulated to further include pharmaceutically acceptable diluents, carriers, excipients, buffers, salts, stabilizers, preservatives, surfactants, cryoprotective agents, etc. Specifically, the carrier may be, for example, a colloidal suspension, powder, saline solution, lipids, liposomes, microspheres, or nano-spherical particles. These may form a complex with or be associated with a delivery means and may be delivered in vivo using a delivery system known in the art, such as lipids, liposomes, microparticles, gold, nanoparticles, polymers, condensation agents, polysaccharides, polyamino acids, dendrimers, saponins, adsorption-enhancing substances, or fatty acids.

The buffer is not particularly limited in type as long as it is known to be used in vaccine formulations, but histidine, citrate, phosphate, succinate, or Hepes (4-(2-hydroxyethyl)-l-piperazineethanesulfonic acid) may be used. In addition, the salt may be selected from the group consisting of magnesium chloride, potassium chloride, sodium chloride, borate chloride, and combinations thereof, and the surfactant may be selected from the group consisting of polysorbate 20, polysorbate 40, polysorbate 60, polysorbate 65, polysorbate 80, polysorbate 85, Triton N-101, Triton X-100, octoxynol 40, nonoxynol-9, triethanolamine, triethanolamine polypeptide oleate, polyoxyethylene-660 hydroxystearate (PEG-15, Solutol H 15), OTG (octylthioglucoside), OG (octylglucoside), NM (nonylmaltoside), LDAO (lauryldimethylamine oxide), DDM (dodecyl beta-D-maltoside), and poloxamer.

The diluent may be a non-aqueous solvent such as propylene glycol, polyethylene glycol, vegetable oils such as olive oil and peanut oil, or an aqueous solvent such as brine (preferably 0.8% brine) or water containing a buffer medium (preferably 0.05M phosphate buffer), and the excipient may be starch, glucose, lactose, sucrose, gelatin, malt, rice, wheat flour, chalk, silica gel, sodium stearate, glycerol monostearate, talc, sodium chloride, anhydrous skim milk, glycerol, propylene glycol, water, ethanol, etc. The stabilizer may be a carbohydrate such as sorbitol, mannitol, starch, sucrose, dextran, glutamate, glucose, etc.; or a protein such as animal protein such as milk powder, serum albumin, casein, plant or microbial protein, etc.; and the above preservative may be thimerosal, methyl oleate, gentamicin, neomycin, nystatin, amphotericin B, tetracycline, penicillin, streptomycin, polymyxin B, etc.

In addition, the vaccine composition of the present invention may further include an adjuvant. The adjuvant refers to a preparation or substance for increasing the clinical efficacy of the vaccine and may be one or more selected from the group consisting of, for example, complete Freund, incomplete Freund, saponin, gel-like aluminum adjuvants, surface active substances (e.g., lysolecithin, fluron glycol, polyanionic, peptide, oil or hydrocarbon emulsion, etc.), vegetable oils (cottonseed oil, peanut oil, corn oil, etc.), vitamin E acetate, squalene, GLA-SE, muramyl dipeptide, lipopolysaccharide (LPS), Cuyl A, Alum (aluminum salts), Al(OH)3, and alpha-galactosylceramide (α-GC).

The vaccine composition of the present invention is administered in a pharmaceutically effective amount. The "pharmaceutically effective amount" refers to a sufficient amount capable of demonstrating the effect of the vaccine, such as significantly reducing the probability of infection with a specific pathogen or disease, or the severity of the disease, and is sufficient to prevent side effects or severe or excessive immune responses. The level of the effective dose may vary depending on various factors, including the disorder to be treated, the severity of the disorder, the activity of the vaccine material, the route of administration, the rate of protein clearance, the duration of treatment, substances combined with or used simultaneously with the vaccine composition, the age, weight, gender, dietary habits, general health status, and factors known in the medical field.

The vaccine composition of the present invention may be administered to an individual prophylactically before infection with the relevant pathogen or before the onset of the disease, and may also be administered therapeutically after infection with the relevant pathogen or after the onset of the disease.

Meanwhile, the vaccine composition of the present invention may be administered by an appropriate method depending on the purpose, such as intramuscular injection, subcutaneous injection, intradermal injection, intraperitoneal injection, nasal administration, oral administration, transdermal administration, or oral administration.

Hereinafter, the present invention will be explained in detail through examples.

However, the following examples are intended to specifically illustrate the present invention, and the content of the present invention is not limited by the following examples.

### [Example 1]

### Design of mRNA structures for various protein expression and measurement of protein expression capacity

In order to design mRNA structures capable of most effectively expressing various target proteins, GFP (Green fluorescent protein), CAR (Chimeric antigen receptor), and SARS-CoV-2 spike protein were selected as target proteins, and various mRNA structures were designed to measure mRNA and protein expression capabilities.

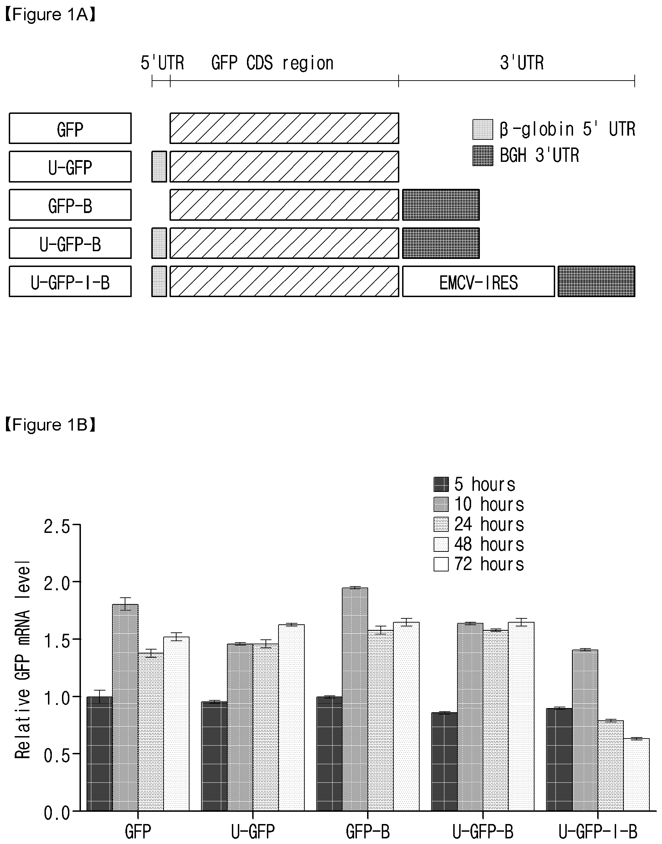

First, GFP was selected as the target protein, and five mRNA structures were constructed by combining a region encoding GFP (SEQ ID NO: 10) with a 5'-β-globin UTR (untranslated region) region upstream of the region (SEQ ID NO: 8), and a BGH (bovine growth hormone) UTR region (SEQ ID NO: 9) and an Encephalomyocarditis virus (EMCV)-derived IRES (Internal ribosome entry site) region (SEQ ID NO: 13) downstream thereof(FIG. 1A).

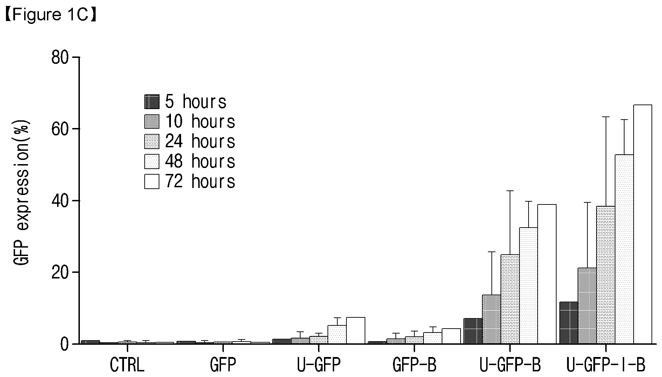

Subsequently, GFP mRNA and protein expression levels were measured using the five structures, and the GFP protein expression was found to be highest in the structure (U-GFP-I-B) containing a 5'-β-globin UTR region upstream of the GFP sequence and an IRES-BGH region downstream thereof (FIGS. 1B and 1C).

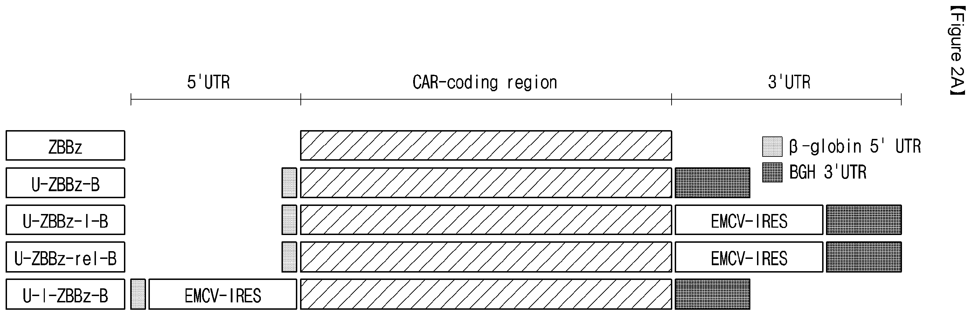

Next, CAR protein was selected as the target protein, and five mRNA structures were constructed by combining the region encoding the CAR protein (SEQ ID NO: 11) with a 5'-β-globin UTR region upstream of the region (SEQ ID NO: 8), and a BGH UTR region (SEQ ID NO: 9) and an EMCV-derived IRES region (SEQ ID NO: 13) downstream thereof (FIG. 2A).

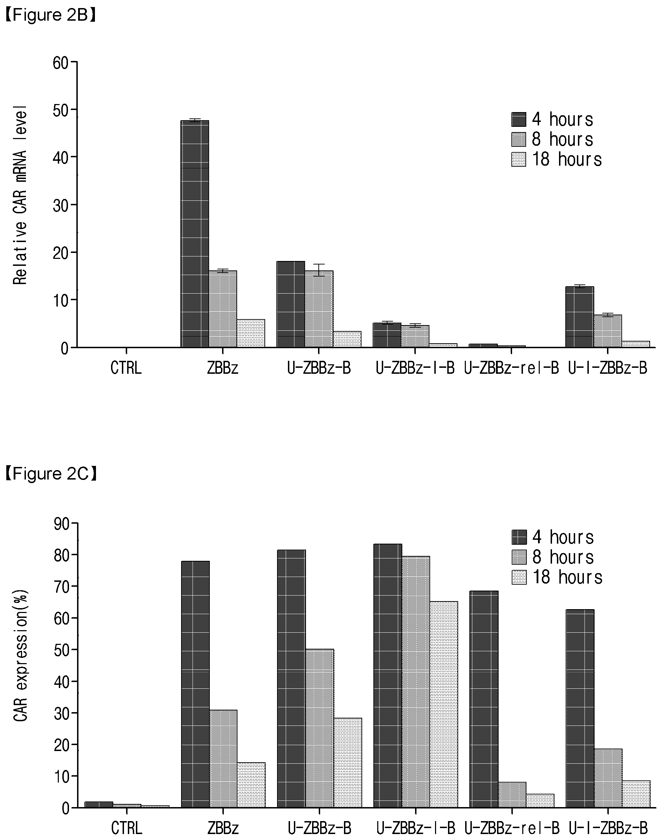

Subsequently, CAR mRNA and protein expression levels were measured using the five structures, and the CAR protein expression was found to be highest in the structure (U-ZBBz-I-B) containing a 5'-β-globin UTR region upstream of the CAR protein sequence and an IRES-BGH region downstream thereof (FIGS. 2B and 2C).

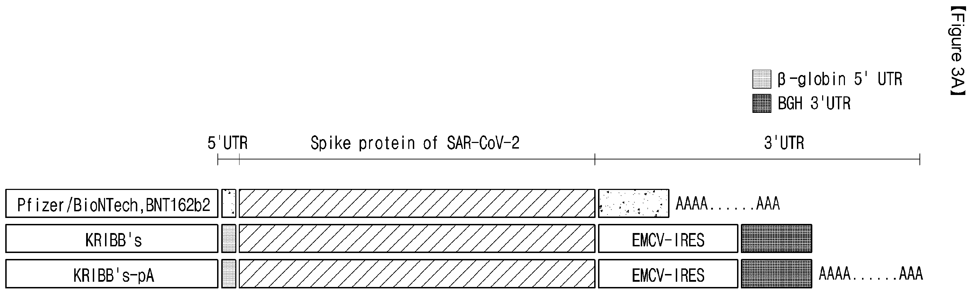

Finally, the spike protein of SARS-CoV-2 was selected as the target protein, and mRNA structures were constructed to include a region encoding the spike protein (SEQ ID NO: 12), a 5'-β-globin UTR region upstream of said region (SEQ ID NO: 8), and a BGH UTR region (SEQ ID NO: 9) and an EMCV-derived IRES region (SEQ ID NO: 13) downstream thereof. mRNA structures were constructed to further include a poly-A region in the structures (FIG. 3A).

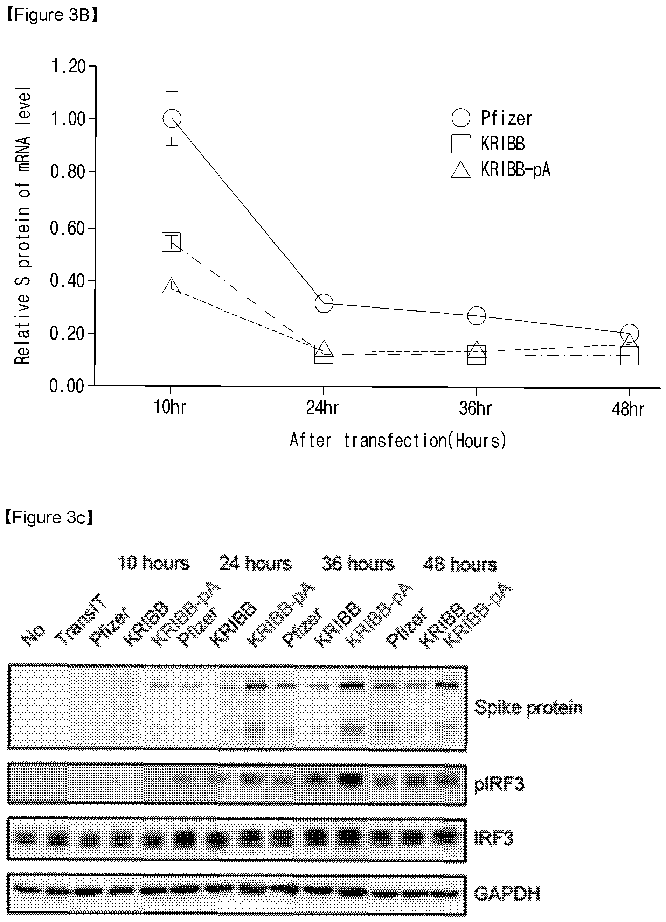

Subsequently, the expression levels of SARS-CoV-2 spike mRNA and protein from the two structures and BNT162b2, a COVID-19 mRNA vaccine sold by Pfizer company, were compared. As a result, the spike protein expression of the structure further containing a poly-A region (KRIBB's-pA) was found to be the highest (FIGS. 3B and 3C).

mRNA expression levels were determined by lysing cells transformed with the constructed mRNA structures and extracting the RNA present in the cells. The extracted RNA was used to synthesize cDNA containing only the RNA with poly A using oligo dT primers, and then the amount of cDNA was measured through a PCR reaction using primers specific to the transformed mRNA structures. Based on this, the amount of RNA was relatively calculated.

In addition, the method for measuring protein expression level is divided according to the type of target protein. First, for mRNA structures encoding GFP and CAR target proteins, protein expression was measured using a flow cytometer after recovering the transformed cells. Meanwhile, for the SARS-CoV-2 spike protein, the protein present in the cells was extracted by lysing the transformed cells, and then the protein expression level was measured through a Western blot experiment using an antibody that can specifically bind to the spike protein.

Through the above experiment, it was confirmed that an mRNA structure containing a region encoding a target protein, a 5'-β-globin UTR region (SEQ ID NO: 8) upstream of the region, and a BGH UTR region (SEQ ID NO: 9) and an EMCV-derived IRES region (SEQ ID NO: 13) downstream of the region can effectively express various target proteins, such as GFP, CAR, and SARS-CoV-2 spike proteins.

### [Example 2]

### Production of optimized mRNA structures through modification of the IRES region

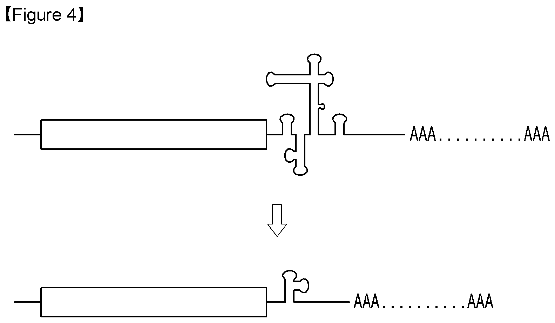

In order to construct mRNA structures that are more optimized for protein expression by increasing the stability and protein expression efficiency of the mRNA structures constructed in Example 1 and reducing immunogenicity, mRNA structures containing RNA structures containing artificially constructed modified IRES sequences (miniIRES; mIRES) were constructed by simplifying the complex secondary structure of the entire IRES sequence (FIG. 4).

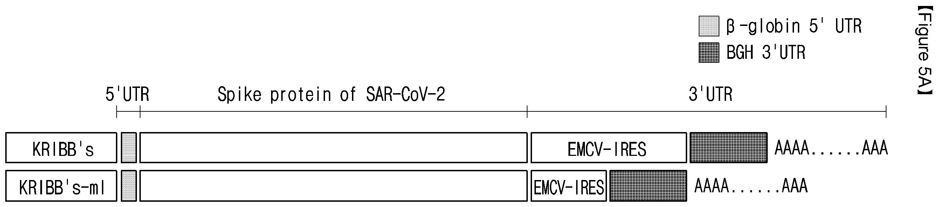

Subsequently, SARS-CoV-2 spike protein was selected as the target protein, and a structure containing a region encoding the spike protein, a 5'-β-globin UTR region (SEQ ID NO: 8) upstream of the region, a BGH UTR region (SEQ ID NO: 9), an EMCV-derived IRES region (SEQ ID NO: 13), and a poly-A region downstream of the region(KRIBB'S), and a structure containing a modified EMCV IRES region (SEQ ID NO: 1) were constructed (KRIBB'S-ml), and the mRNA stability, protein expression efficiency, and immunogenicity of the two structures were compared with those of BNT162b2, a COVID-19 mRNA vaccine sold by Pfizer company (FIG. 5A).

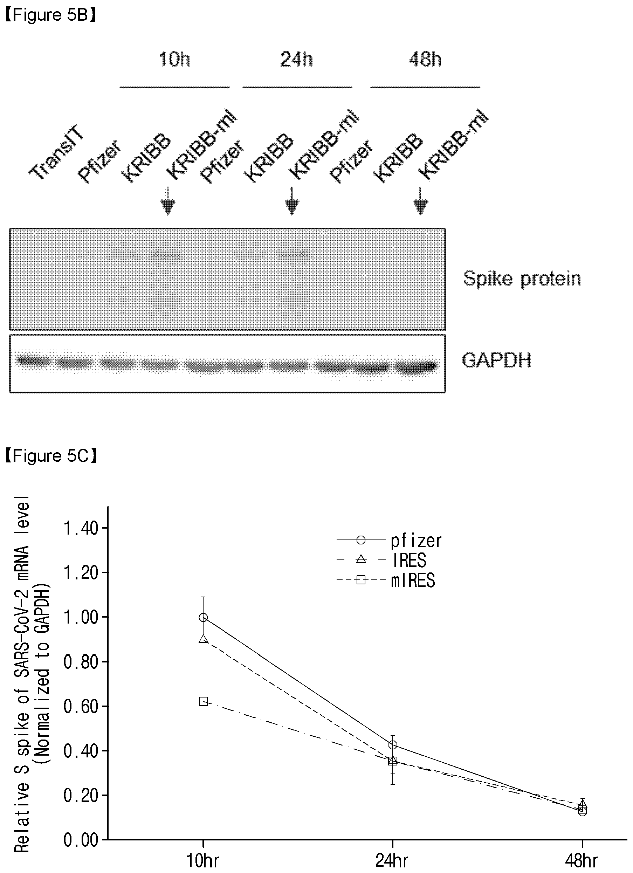

As a result, the spike protein expression level of the structure containing the modified EMCV IRES region (SEQ ID NO: 1) was found to be significantly higher compared to the structure containing the unmodified EMCV IRES region (SEQ ID NO: 13) and BNT161b2 (FIG. 5B). In addition, the mRNA stability of the structure containing the modified EMCV IRES region (SEQ ID NO: 1) was found to be significantly higher compared to the structure containing the unmodified EMCV IRES region (SEQ ID NO: 13) (FIG. 5C).

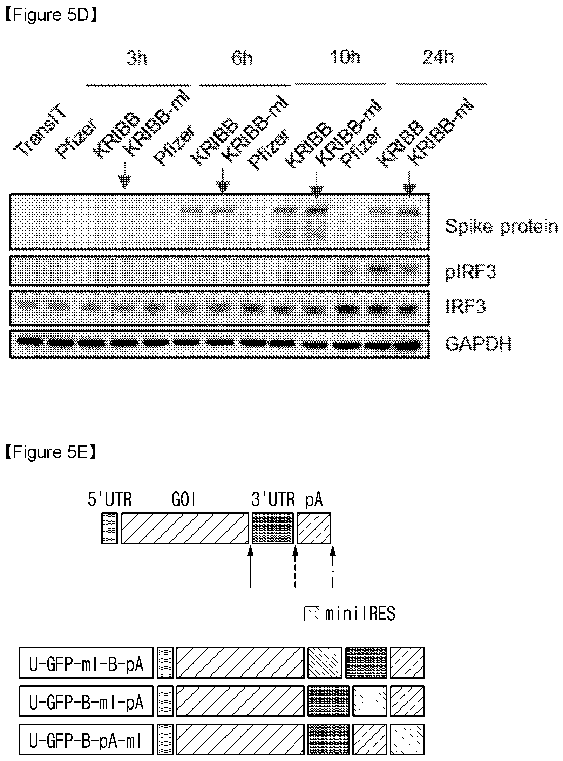

Furthermore, to compare immunogenicity, the expression of phosphor-IRF3, which is an activated form of IRF3 that induces an innate immune response upon viral infection, was measured. The results showed that the expression level of the structure containing the modified EMCV IRES region (SEQ ID NO: 1) was significantly lower than that of the structure containing the unmodified EMCV IRES region (SEQ ID NO: 13) (FIG. 5D).

Meanwhile, to confirm specifically where the modified IRES region should be introduced downstream of the mRNA structure for optimal efficiency, the modified EMCV IRES region (SEQ ID NO: 1) was introduced between the BGH UTR region (SEQ ID NO: 9) and the poly-A region, respectively (FIG. 5E), and then the GFP expression levels and mRNA stability were compared.

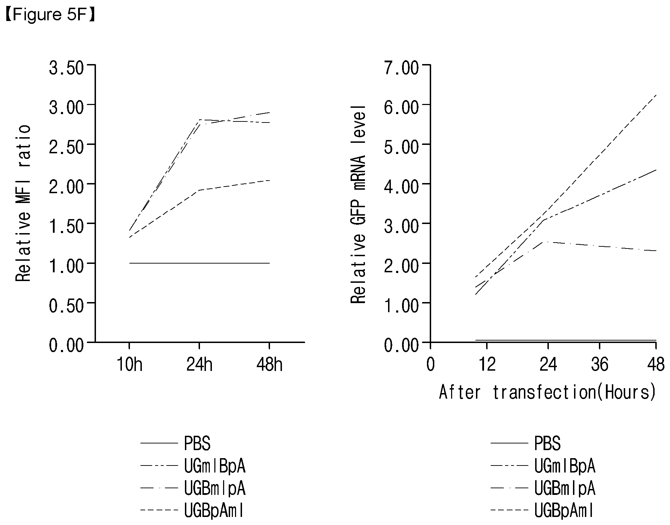

As a result, it was found that when the modified IRES region is located upstream of the BGH UTR region (SEQ ID NO: 9) or between the BGH UTR region (SEQ ID NO: 9) and the poly-A region, GFP is expressed at the same level, but when it is located downstream of the poly-A region, the GFP expression level is significantly reduced (FIG. 5F). Meanwhile, it was confirmed that mRNA stability was superior when the modified IRES region was located upstream of the BGH UTR region (SEQ ID NO: 9) compared to when it was located between the BGH UTR region (SEQ ID NO: 9) and the poly-A region, thus confirming that the location of the modified IRES region upstream of the BGH UTR region (SEQ ID NO: 9) is the best in terms of target protein expression and mRNA stability (FIG. 5F).

Through the above experiment, optimal mRNA structures were designed by simplifying the secondary structure of the IRES region within the mRNA structures, thereby increasing mRNA stability and protein expression efficiency while simultaneously reducing immunogenicity.

### [Example 3]

### Production of mRNA structures modified from various virus-derived IRES regions

Both Examples 1 and 2 above were performed by constructing mRNA structures containing RNA structures obtained from the EMCV-derived IRES region. Accordingly, experiments were conducted to confirm whether the mRNA structures of the present invention can effectively express a target protein even when constructed from an IRES region derived from an RNA virus other than EMCV.

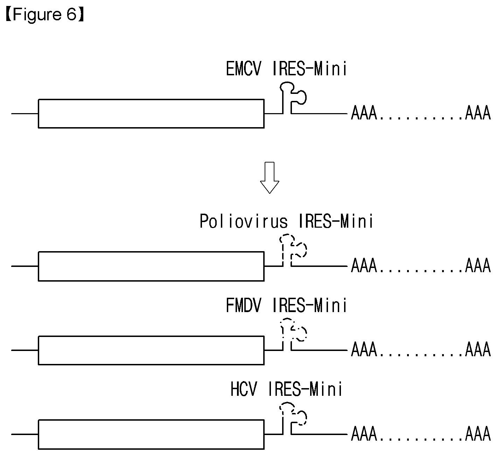

Specifically, in addition to EMCV, the secondary structures of the IRES regions of Poliovirus, Foot-and-mouth disease virus (FMDV), and Hepatitis C virus (HCV) were simplified, and mRNA structures containing RNA structures obtained by simplifying the IRES regions of each virus were constructed (SEQ ID NOs: 2 to 7 and FIG. 6).

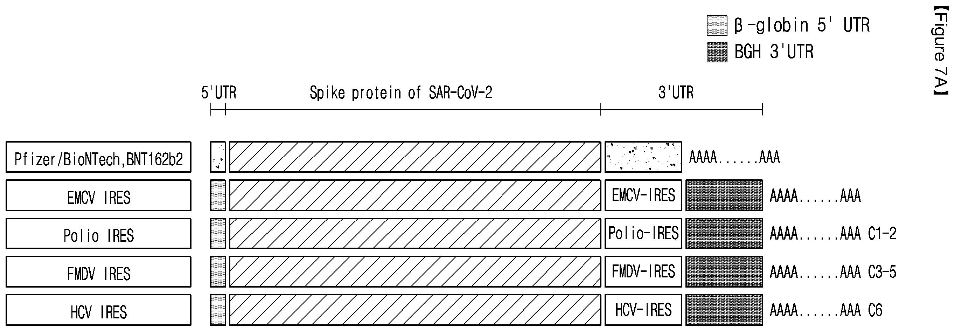

Subsequently, the spike protein of SARS-CoV-2 was selected as the target protein, and mRNA structures containing the spike-encoding region (SEQ ID NO: 12) and the respective RNA virus-derived modified IRES region were constructed. Specifically, a structure containing an EMCV-derived modified IRES region (SEQ ID NO: 1), structures (C1 and C2) containing poliovirus-derived modified IRES regions (SEQ ID NOs: 2 and 3), structures (C3 to C5) containing FMDV-derived modified IRES regions (SEQ ID NOs: 4 to 6), and a structure (C6) containing an HCV-derived modified IRES region (SEQ ID NO: 7) were constructed (FIG. 7A).

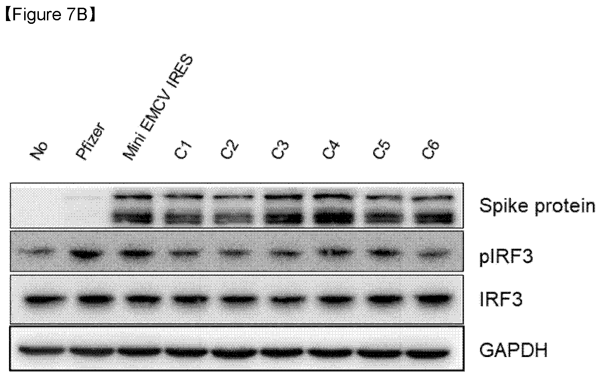

Next, the spike protein expression level was measured using the mRNA structures constructed as described above, and it was confirmed that the spike protein was expressed at a significantly higher amount in all structures than in Pfizer company's BNT162b2 mRNA structure (FIG. 7B).

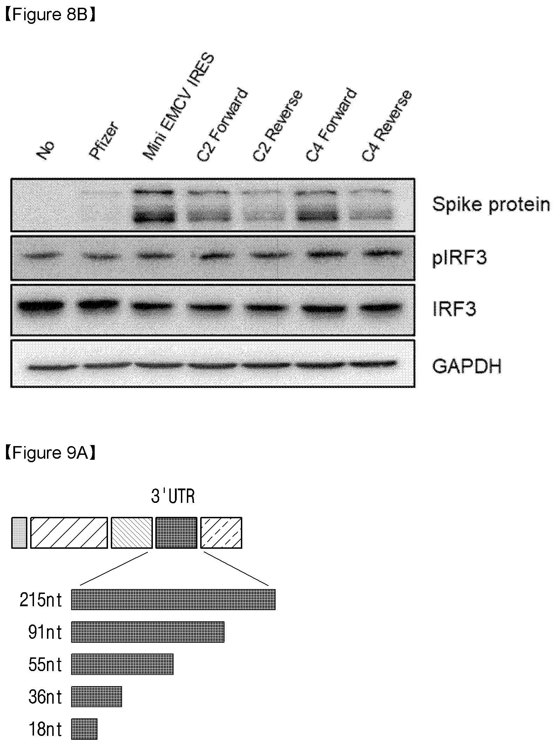

In addition, to investigate the RNA structural specificity of the poliovirus-derived modified IRES region (C2 Forward) and the FMDV-derived modified IRES region (C4 Forward), mRNA structures were designed to include an IRES region with opposite sequences (C2/C4 Reverse), and then spike protein expression levels were measured (FIG. 8A). As a result, it was confirmed that spike protein was expressed in significantly higher amounts in all structures, including the opposite strand, than in Pfizer company's BNT162b2 mRNA structure (FIG. 8B). However, it was confirmed that the spike protein expression levels in the above structures were lower than those in mRNA structures with an EMCV-derived modified IRES region introduced.

Through the above experiments, it was confirmed that the mRNA structures of the present invention can effectively express a target protein even when constructed using the IRES region of various RNA viruses in addition to EMCV, and this was confirmed to be RNA structure-specific.

### [Example 4]

### Production of optimized mRNA structures by adjusting the length of the BGH UTR region

In Examples 1 to 3 above, a nucleotide sequence of SEQ ID NO: 9, consisting of 215 nucleotides, was used as the BGH UTR region. Below, experiments were conducted to derive the BGT UTR region with the highest target protein expression ability by comparing target protein expression levels after varying the length of the BGH UTR region.

First, BGH UTR regions consisting of 91, 55, 36, and 18 nucleotides were constructed from the BGH UTR region of SEQ ID NO: 9, which consists of 215 nucleotides (SEQ ID NOs: 14 to 17), respectively, and mRNA structures containing these were constructed (FIG. 9A).

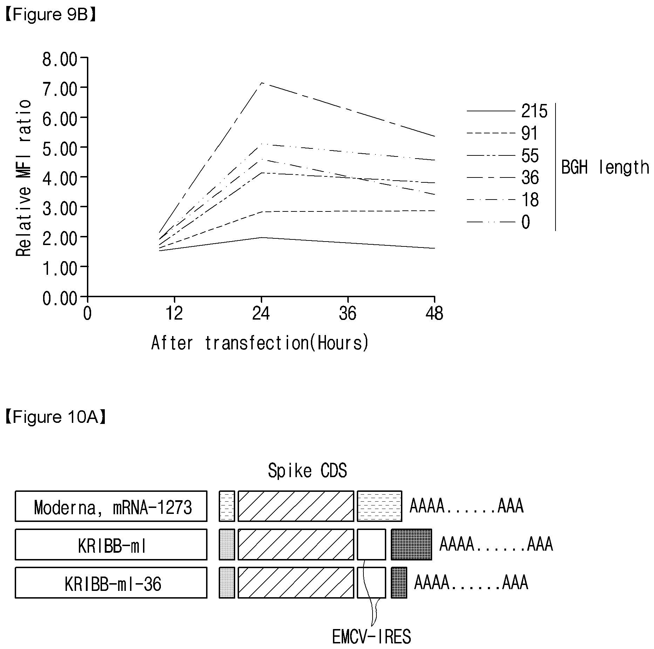

Subsequently, the GFP expression capacity of each mRNA structure was measured, and it was found that the GFP expression level of the mRNA structure containing a BGH UTR region (SEQ ID NO: 16) consisting of 36 nucleotides was significantly higher than that of the existing BGH UTR region of SEQ ID NO: 9, thereby deriving an optimized BGH UTR region sequence capable of most effectively expressing the target protein (FIG. 9B).

Next, an mRNA structure containing a spike-encoding region (SEQ ID NO: 12) and a modified EMCV IRES region (SEQ ID NO: 1) located upstream of a BGH UTR region (SEQ ID NO: 9), and an mRNA structure in which a modified EMCV IRES region (SEQ ID NO: 1) located upstream of an optimized BGH UTR region (SEQ ID NO: 16) consisting of 36 nucleotides were constructed (FIG. 10A), and their target protein expression capabilities were compared with Moderna company's mRNA-1273 structure.

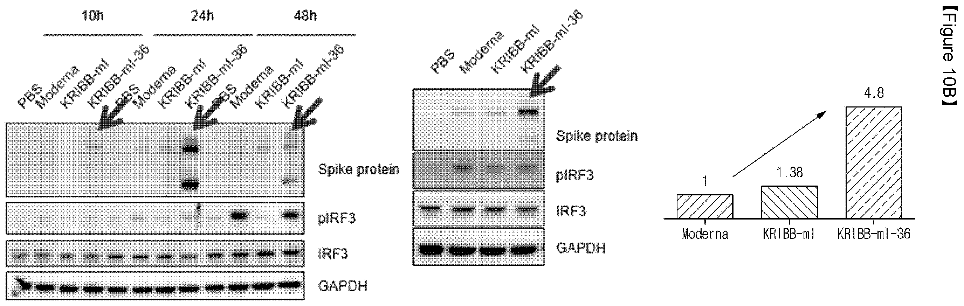

As a result, both mRNA structures showed significantly higher spike protein expression than Moderna company's mRNA-1273 structure, and in particular, the spike protein expression level of the mRNA structure containing the optimized BGH UTR region (SEQ ID NO: 16) was found to be about 4.8 times higher than that of Moderna company's structure (FIG. 10B).

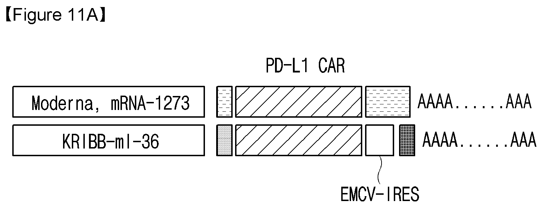

Furthermore, after constructing an mRNA structure in which a CAR protein was selected as the target protein and included a region encoding the CAR protein (SEQ ID NO: 11), a 5'-β-globin UTR region upstream of said region (SEQ ID NO: 8), and a modified EMCV IRES region (SEQ ID NO: 1) and an optimized BGH UTR region consisting of 36 nucleotides (SEQ ID NO: 16) downstream thereof, the protein expression capacity was compared with Moderna company's mRNA-1273 structure (FIG. 11A).

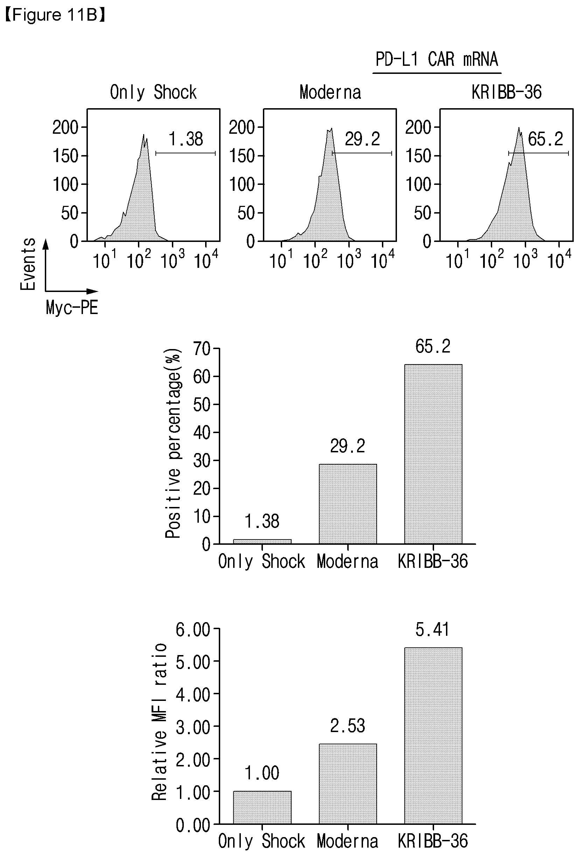

As a result, as with the case of spike protein mentioned earlier, the CAR protein expression level of the mRNA structure containing the modified EMCV IRES region (SEQ ID NO: 1) and the optimized BGH UTR region (SEQ ID NO: 16) was found to be about 2 to 3 times higher than that of Moderna company's structure (FIG. 11B).

Through the above experiment, mRNA structures with optimized protein expression efficiency were designed by simplifying the secondary structure of the IRES region, positioning the IRES region, and changing the length of the BGH UTR region.

### [Example 5]

### Introduction of mRNA structure using various cells and methods

In this Example, an experiment was conducted to verify whether the mRNA structure of the present invention can effectively express a target protein even when introduced into various cells and by various methods.

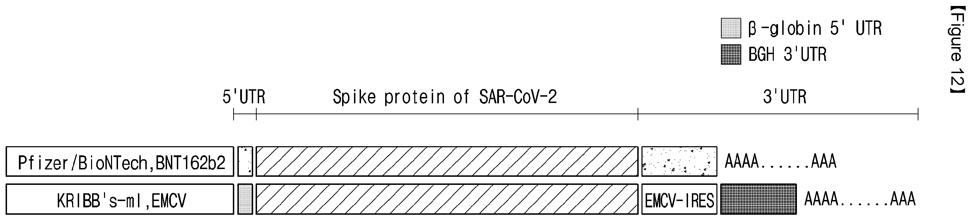

Accordingly, the spike protein of SARS-CoV-2 was first selected as the target protein, and a structure was constructed containing a region encoding the spike protein (SEQ ID NO: 12) and an EMCV-derived modified IRES region (SEQ ID NO: 1) (FIG. 12).

Subsequently, the above structure was introduced into epithelial cells, HEK-293T cells or muscle cells, c2c12 cells, and the expression level of the spike protein was compared with the expression level in cells introduced with Pfizer company's COVID-19 mRNA vaccine, BNT162b2.

Subsequently, the above structure was introduced into epithelial cells, HEK-293T cells or muscle cells, c2c12 using a liposome-based transformation method, and the expression level of the spike protein was compared with the expression level in cells introduced with Pfizer company's COVID-19 mRNA vaccine BNT162b2.

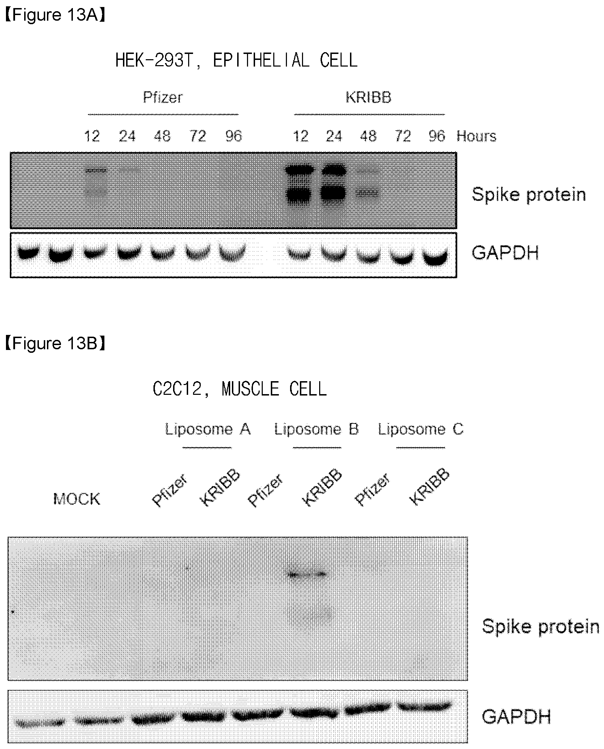

As a result, the spike protein expression level in HEK-293T cells introduced with the mRNA structure of the present invention was found to be significantly higher than the expression level in BNT162b2-introduced cells, and the spike protein expression level in c2c12 cells introduced with the mRNA structure of the present invention was also found to be significantly higher than the expression level in BNT162b2-introduced cells (FIGS. 13A and 13B).

Next, the above structure was introduced into HEK-293T cells using liposomes or LNPs (Lipid nanoparticles), and the expression level of the spike protein was compared with the expression level in cells introduced with BNT162b2.

For liposomes, mRNA-liposome complexes were synthesized using the "TransIT-X2 Dynamic Delivery System" sold by "Mirus" company and administered into cells. For LNP, an LNP mixture was formed by mixing 50% ALC-0315, 10% DSPC, 38.5% Cholesterol, and 1.5% DMG-PEG, and this was mixed with mRNA to synthesize mRNA-LNP complexes using a microfluidic device and administered into cells.

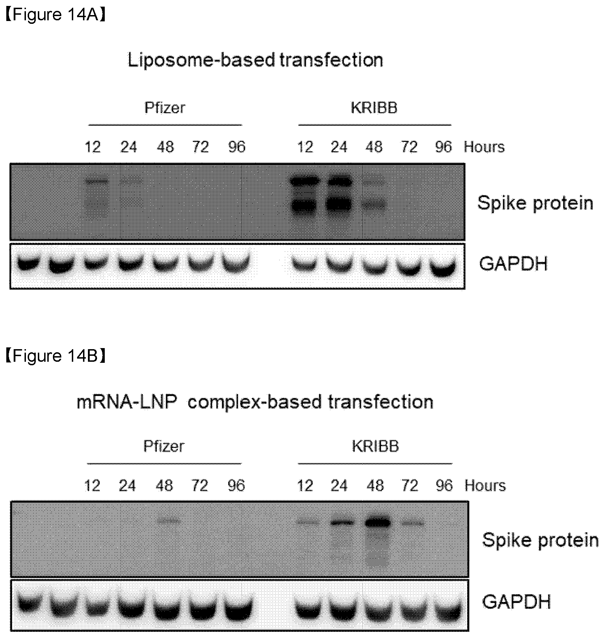

As a result, the expression level of spike protein in HEK-293T cells into which the mRNA structure of the present invention was introduced using liposomes was found to be significantly higher than the expression level in BNT162b2-introduced cells, and the expression level of spike protein in HEK-293T cells into which the mRNA structure of the present invention was introduced using LNP was also found to be significantly higher than the expression level in BNT162b2-introduced cells (FIGS. 14A and 14B).

Through the above experiments, it was confirmed that the mRNA structures of the present invention can effectively express a target protein even when introduced into various cells, such as epithelial cells and muscle cells, and also when introduced into cells by various methods, such as liposomes and LNPs.

Therefore, since the mRNA structures of the present invention can stably and efficiently express various target proteins for a long period of time within various cells, they are expected to be effectively utilized in various application fields related to target protein expression, such as anticancer immunotherapy and vaccine development.

Although representative embodiments of the present invention have been described above by way of example, the scope of the present invention is not limited only to such specific embodiments, and those skilled in the art will be able to make appropriate modifications within the scope described in the claims of the present application.

## Claims

1. An RNA structure for improving the efficiency of non-naturally occurring protein translation, comprising: a nucleic acid sequence that comprises two or more stem structures; and two or more loop structures, wherein the nucleic acid sequence is a virus-derived internal ribosome entry site(IRES).

2. The RNA structure of claim 1, wherein the virus is Encephalomyocarditis virus(EMCV), Poliovirus, Foot-and-mouth disease virus (FMDV), or Hepatitis C virus(HCV).

3. The RNA structure of claim 1, wherein the nucleic acid sequence forms 2 to 8 stem structures; and 2 to 8 loop structures.

4. The RNA structure of claim 1, wherein the RNA structure binds to eukaryotic translation initiation factor(eIF).

5. The RNA structure of claim 1, wherein the nucleic acid sequence is composed of any one of nucleotide sequences of SEQ ID NOs: 1 to 7.

6. An mRNA structure, comprising (a) a region encoding a target protein or peptide, and (b) the RNA structure of claim 1 downstream of the region encoding the target protein or peptide.

7. The mRNA structure of claim 6, further comprising 3'-UTR region of bovine growth hormone(BGH) (b) downstream of the region encoding the target protein or peptide.

8. The mRNA structure of claim 7, wherein the 3'-UTR region of the BGH is composed of any one of the nucleotide sequences of SEQ ID NO: 9 and SEQ ID NOs: 14 to 17.

9. The mRNA structure of claim 6, wherein the RNA structure of claim 1 is included (a) downstream of the region encoding the target protein or peptide, and upstream of the 3'-UTR region of the BGH.

10. The mRNA structure of claim 6, wherein the target protein or peptide is for prevention or treatment of disease.

11. The mRNA structure of claim 10, wherein the target protein or peptide is at least one selected from the group consisting of antigens, viral proteins, enzymes, chimeric antigen receptors (CARs), cell receptors, and serum proteins.

12. A recombinant vector, comprising a nucleotide sequence corresponding to the nucleotide sequence of the mRNA structure of claim 6 or a DNA nucleotide sequence complementary thereto.

13. A transformant in which the mRNA structure of claim 6 is introduced into a host cell.

14. A pharmaceutical composition for prevention or treatment of infectious diseases comprising the mRNA structure of claim 6; the recombinant vector of claim 12; or the transformant of claim 13.

15. The pharmaceutical composition for the prevention or treatment of infectious diseases of claim 14, wherein the infectious diseases are at least one selected from the group consisting of sepsis, septic shock, Severe Acute Respiratory Syndrome Coronavirus (SARS-CoV) infection, Middle East Respiratory Syndrome (MERS), salmonellosis, food poisoning, typhoid fever, paratyphoid fever, Systemic Inflammatory Response Syndrome (SIRS), Multiple Organ Dysfunction Syndrome (MODS), pneumonia, pulmonary tuberculosis, tuberculosis, common cold, influenza, respiratory infection, rhinitis, nasopharyngitis, otitis media, bronchitis, lymphadenitis, parotitis, lymphadenitis, cheilitis, stomatitis, arthritis, myositis, dermatitis, vasculitis, gingivitis, periodontitis, keratitis, conjunctivitis, wound infection, peritonitis, hepatitis, osteomyelitis, cellulitis, meningitis, encephalitis, brain abscess, encephalomyelitis, cerebral meningitis, osteomyelitis, nephritis, carditis, endocarditis, enteritis, gastritis, esophagitis, duodenitis, colitis, urinary tractitis, cystitis, vaginitis, cervicitis, salpingitis, infectious erythema, bacterial dysentery, abscess and ulcer, bacteremia, diarrhea, dysentery, gastritis, gastroenteritis, genitourinary abscess, infection of open wounds or wounds, suppurative inflammation, abscess, boil, pyoderma, impetigo, folliculitis, cellulitis, postoperative wound infection, skin laceration syndrome, skin burn syndrome, thrombotic thrombocytopenia, hemolytic uremic syndrome, renal failure, pyelonephritis, glomerulonephritis, nervous system abscess, otitis media, sinusitis, pharyngitis, tonsillitis, mastoiditis, cellulitis, odontogenic infection, dacryocystitis, pleuritis, abdominal abscess, liver abscess, cholecystitis, splenic abscess, pericarditis, myocarditis, placentaitis, amnioticitis, mastitis, puerperal fever, toxic shock syndrome, Lyme disease, gas gangrene, atherosclerosis, Mycobacterium avium syndrome (MAC), enterohemorrhagic E. coli (EHEC) infection, enteropathogenic E. coli (EPEC) infection, enteroinvasive E. coli (EIEC) infection, methicillin-resistant Staphylococcus aureus (MRSA) infection, vancomycin-resistant Staphylococcus aureus (VRSA) infection, and listerosis.

16. A pharmaceutical composition for prevention or treatment of non-infectious diseases, comprising the mRNA structure of claim 6; the recombinant vector of claim 12; or the transformant of claim 13.

17. The pharmaceutical composition for the prevention or treatment of non-infectious diseases of claim 16, wherein the non-infectious diseases are at least one selected from the group consisting of cancer, autoimmune diseases, non-infectious inflammatory diseases, neurodegenerative diseases, heart diseases, stroke, diabetes, metabolic diseases, chronic kidney diseases, and chronic respiratory diseases.

18. A vaccine composition, comprising the mRNA structure of claim 6; the recombinant vector of claim 12; or the transformant of claim 13.
